# EUROPEAN PATENT APPLICATION

(11) **EP 2 172 237 A1**
(43) Date of publication of application: **07.04.2010**
(21) Application number: 09169115.4
(22) Date of filing: 01.09.2009
(51) Int. Cl.: A61M 5/142, A61M 5/14

(54) **Electronic tag system**

(30) Priority: 12.09.2008 US 283493
(71) Applicant: Delphi Technologies, Inc., Troy, MI 48007 (US)
(72) Inventor: Nelson, James E., North Branch, MI 48461 (US); Voltenburg, Jr., Robert R., Davison, MI 48423 (US)
(74) Representative: Denton, Michael John

(57) **Abstract**

An electronic tag system (10) includes a medical device (12) having a base portion (14), a removable portion (16), and an electronic tag (18) operatively connected to the removable portion. The electronic tag includes data related to at least a medicinal infusion process. The system further includes a control element (20) operatively connected to the base portion. The control element includes a reading capability for reading the data from the electronic tag and a writing capability for writing new data to the electronic tag.

## Description

### BACKGROUND OF THE INVENTION

The present disclosure relates generally to electronic tag systems and, more particularly, to an electronic tag system for a medical device.

Medical infusion devices are often used to infuse a medicament to a subject such as, e.g., a human being or other animal. These devices typically include a reusable unit or portion such as, e.g., an infusion pump, having a removable and disposable unit operatively connected thereto. The disposable unit includes a flexible tube connected thereto and configured to have the medicament flow through it for delivery thereof to the subject during an infusion process. When the infusion process is complete, the disposable unit is usually thrown away.

In some instances, the disposable unit is used for several infusion processes for a single, particular subject. In at least these instances, information regarding the usage time, the amount of medicament infused, infusion process parameters, manufacturing data, shelf-life of the disposable unit, and/or the like may be useful. Such information may be stored in an electronic tag (referred to herein as an "e-tag") that may be read from a control device associated with the reusable unit of the medical device.

### SUMMARY OF THE INVENTION

An electronic tag system includes a medical device having a base portion, a removable portion, and an electronic tag operatively connected to the removable portion. The electronic tag includes data related to a medicinal infusion process. The system further includes a control element operatively connected to the base portion. The control element includes a reading capability for reading the data from the electronic tag and a writing capability for writing new data to the electronic tag.

### BRIEF DESCRIPTION OF THE DRAWINGS

Features and advantages of the present disclosure will become apparent by reference to the following detailed description and the drawings, in which like reference numerals correspond to similar, though perhaps not identical components. For the sake of brevity, reference numerals or features having a previously described function may or may not be described in connection with other drawings in which they appear.

Fig. 1 is a semi-schematic, perspective, side view of an embodiment of an electronic tag system including an infusion device and an electronic tag; and

Fig. 2 is a diagram depicting an embodiment of a method of tracking a removable infusion set for an infusion pump.

### DESCRIPTION OF THE PREFERRED EMBODIMENTS

Embodiment(s) of the e-tag system as disclosed herein includes an e-tag, having data stored therein, operatively connected to a disposable unit of a medical device. The e-tag is advantageously configured so that a control element associated with a reusable portion of the medical device may read data from the e-tag, as well as write data to the e-tag. Further, the e-tag system advantageously includes a communication means allowing the control element to communicate with the e-tag and request, e.g., that the e-tag send specific data, that the e-tag perform a specific function, and/or the like. In instances where the disposable unit is used in multiple infusion processes with different reusable units, the data may be stored in the e-tag for each infusion process. This data may advantageously be used to track to the disposable unit throughout its operable life. Additionally, some of the data stored in the e-tag may be used to determine, e.g., whether the disposable unit is authentic, sterile, not used past its expiration date, the infusion parameters and protocol, and/or the like.

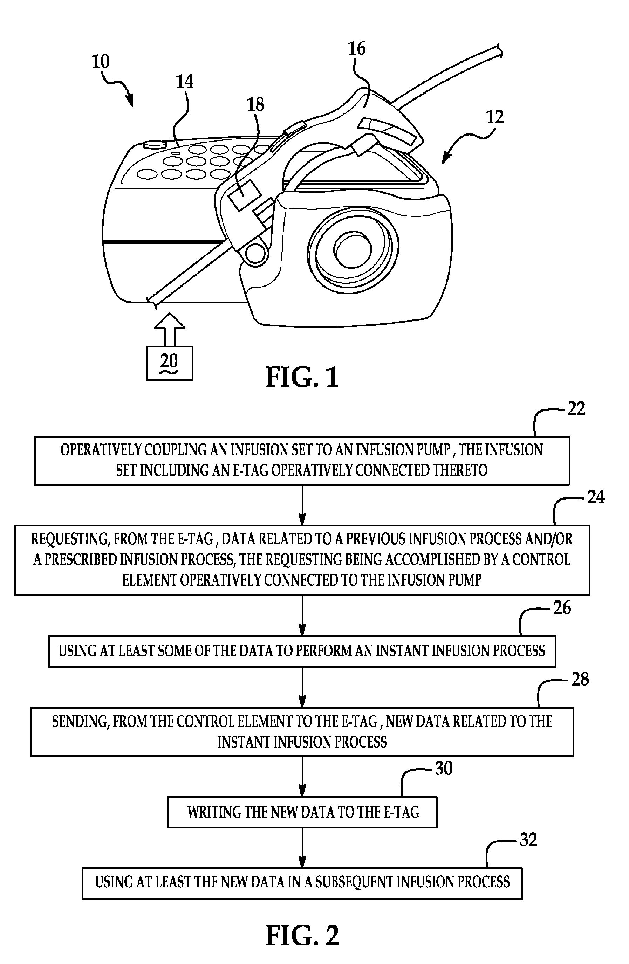

With reference now to Fig. 1, an electronic tag (e-tag) system 10 includes a medical device 12 having a base portion 14 and a removable portion 16. In an example, as shown in Fig. 1, the medical device 12 is an infusion device configured to infuse a medicament to a subject. The base portion 14 is an infusion pump, and the removable portion 16 is an infusion set. Details of the medical device depicted in Fig. 1 may be found in commonly owned U.S. Patent Application Ser. No. 11/862,302 (Attorney Docket No. DP-315954), which is incorporated herein by reference in its entirety. It is to be understood, however, that the infusion device 12 depicted in Fig. 1 is one of many possible variations of infusion devices that fall within the spirit and scope of the instant disclosure. It is further to be understood that other variations of medical devices may also be used herein such as, e.g., syringe devices, dialysis sets, infusion filters, respiratory heat and/or moisture exchange devices, respiratory heat and/or moisture exchange filters, various gas sensors for anesthesia (such as, e.g., a micro fuel cell oxygen sensor), or the like.

An e-tag 18 (schematically shown in Fig. 1) is operatively connected to the infusion set 16. In a non-limiting example, the e-tag 18 is removably attached to the infusion set 16 so that the e-tag 18 may be removed from the infusion set 16 when the infusion set 16 is disposed of. In this example, the e-tag 18 may be placed in the subject's medical records. In another non-limiting example, the e-tag 18 is actually affixed to the infusion set 16 so that it cannot be removed. In this example, the e-tag 18 is disposed of when the infusion set 16 is disposed of.

The e-tag 18 is configured to have data stored or otherwise programmed therein. The data may be classified as either accessible data or limited access data. For example, accessible data may include infusion parameters and/or a protocol for performing an instant infusion process. Such parameters include, but are not limited to, a dosage, a bolus, an infusion time interval, a volume of infusate to be administered, and/or the like, and/or combinations thereof. These parameters are often selected based on a prescription made by the subject's physician.

In some instances, the accessible data may further include information relating to the identity of the subject. For example, the subject's name, address, age, weight, and/or other similar information may be included so that, prior to starting an infusion process, the information may be accessed to determine whether the prescription is properly matched with the instant subject. If a match exists, then the infusion process may proceed.

Limited access data is specific data to which authorized persons have access. Limited access data is also specific data that may be accessed by those medical devices that are used solely for internal medical processes. Such data is generally considered to be data that is not intended to be changed, deleted, copied, and/or the like. Non-limiting examples of limited access data include part numbers, serial numbers, lot codes, date codes, manufacturing information, encryption keys, and/or the like, and/or combinations thereof. Another non-limiting example of limited access data includes information protected by the HIPAA (such as, e.g., personal information related to the subject). This information is generally protected so that the information cannot be recovered from, for example, an already discarded removable device by unauthorized third parties.

The infusion pump (i.e., the base portion) 14 includes a control element 20 (also schematically shown in Fig. 1) operatively connected thereto. The control element 20 includes a communication means allowing the infusion pump 14 (through the control element 20) to communicate or interact with the e-tag 18 when the infusion set 16 is coupled to the infusion pump 14. The control element 20 further includes a reading capability for reading data from the e-tag 18 and a writing capability for writing new data to the e-tag 18.

In an embodiment, the communication means is a wired electrical connection established between the control element 20 and the e-tag 18. In order to accomplish the wired connection, the e-tag 18 may, in an example, be configured as a smart card including one or more electrical connection pins or pads, as a fob including an electrical connector, and/or the like. The e-tag 18 may otherwise be configured, in other non-limiting examples, as chip scale packages, die on boards, flip chips, and/or other similar custom packaging or bare board modules.

In another embodiment, the communication means is a wireless connection established between the control element 20 and the e-tag 18. In order to accomplish this connection, the e-tag 18 may be configured, e.g., as a transponder, where communication is accomplished via modulated electromagnetic fields. The transponder may be powered by an internal power source such as, e.g., a battery, or may be derived by rectification of the received signal field. Alternative methods of powering the transponder include, but are not limited to, magnetic induction, optical powering means, via a capacitor, or by rectification of a power transmission separate from the wireless connection means. In another example, wireless communication between the e-tag 18 and the control element 20 may also be accomplished using optical and/or magnetic signals. In this embodiment, the e-tag 18 configured for wireless communication may be provided in the form of a key, a fob, a capsule, a card, a clip, a module, or the like.

In an embodiment, the infusion pump 14 may include a sensor or other means to sense a connection between the infusion set 16 (including the e-tag 18) and the pump 14 when infusion set 16 and the pump 16 are connected or paired. This sensor is generally configured to detect the connection over a relatively short range so that the infusion pump 14 can determine whether or not a particular infusion set 16 is actually installed in the infusion pump 14. In another embodiment, the pump 14 may include a magnetic, mechanical, or optical sensor for determine the proximity of the infusion set 16 to the pump 14 for determining whether the appropriate infusion set 16 is paired with the pump 14. In yet another embodiment, pairing the appropriate infusion set 16 with the pump 14 may be accomplished manually via, e.g., scanning a barcode, entering a code in a keypad, etc. when the infusion set 16 is connected to the pump 14.

The e-tag 18 may be configured to include at least a read-only circuitry allowing the control element 20 to read the data stored in the e-tag 18. In an embodiment, the e-tag 18 includes a read-write circuit allowing the control element 20 to read data stored in the e-tag 18 and to write new data to the e-tag 18. In some instances, the e-tag 18 further includes a resonant circuit that may be probed or energized using an electromagnetic field to also detect the presence of an infusion set appropriate for a particular subject.

Embodiments of the e-tag system 10 described above enables the infusion set 14 to program the infusion pump 16 using the data stored in the e-tag 18 to perform an instant infusion process. As indicated above, this data would include the infusion protocol and parameters as defined by the subject's prescription, as well as infusion results from a previous infusion process (if one was made). During the instant infusion process, the control element 20 generates new data including the infusion protocol and parameters actually performed and/or other results of the instant infusion process. For example, the results of the instant infusion process may include infusion status, timing of the bolus delivered for analgesia, a quantity of the bolus delivered for analgesia, partial infusions, piggyback infusions, substances delivered during the infusion process, a quantity of the substance delivered, a time course of infusion performed such as, e.g., start and stop times, faults, noted malfunctions (e.g., occlusions detects and air-in-line/pressure faults), and/or the like, and/or combinations thereof. The new data may also include the infusion protocol and parameters, as defined by the subject's prescription or by the instant infusion process (in the event that the prescription was changed by authorized personnel) for a subsequent infusion process (if one will be made).

When the infusion set 14 is coupled to the infusion pump 16, the e-tag 18 is connected (either via a wired connection or a wireless connection) with the control element 20 so that the control element 20 is able to communicate with the e-tag 18. Such communication allows the control element 20 to 1) make a request from the e-tag 18 prior to the instant infusion process, or 2) send new data generated from the instant infusion process to the e-tag 18.

In an embodiment, the control element 20 requests at least some of the data stored in the e-tag 18. In another embodiment, the control element 20 requests that the e-tag 18 perform a function or activity. For example, the control element 20 may request that the e-tag 18 perform an encryption of the data stored therein, a decryption of the data (if the data is already encrypted), to send the data, to send a signal representing the data, to store the new data, to store a program, and/or the like, and/or combinations thereof. If it is determined that the control element 20 has permission to access the data stored in the e-tag 18 or has permission to request that the e-tag 18 perform a function, the e-tag 18 responds to the request.

For example, the e-tag 18 may include an authentication system. The authentication system may include, e.g., hardware keys, software or firmware keys, passwords, and/or the like, and/or combinations thereof. The response may be in the form of performing the function requested and/or sending the data requested. It is to be understood that the data may be sent in a non-encrypted form, in an encrypted form, or as a signal representing the data.

If the infusion set 14 including the e-tag 18 is used for a single infusion process, the infusion set 14 may be appropriately disposed of along with the e-tag 18 after the infusion process is complete. In some instances, the e-tag 18 may be removed from the infusion set 14, prior to disposal thereof, and the data stored on the e-tag 18 may be included in the subject's medical records. The e-tag 18 may thereafter be appropriately disposed of as well, if desired.

In instances where the subject uses the infusion set 16 more than once, the data stored on the e-tag 18 may be used to track the infusion set 16. Tracking is advantageous as a safety measure to ensure that 1) the infusion set is used for the intended subject, and 2) the proper infusion protocol and parameters are applied during the infusion process for each infusion pump that the infusion set is coupled to. Specifically, data may be obtained and stored in the e-tag 18 for each infusion process that the infusion set 16 is used and a review of the data reveals whether or not the infusion protocol and/or parameters applied for each infusion process was appropriate.

In an embodiment, the infusion protocol and/or parameters for a particular infusion may be changed without having to change the infusion set 16. In this embodiment, the infusion pump 14 may be configured to determine if the new infusion protocol and/or parameters are within acceptable limits. This may be accomplished by checking the new infusion protocol and/or parameters with, e.g., an internally-stored drug library.

A diagram of an embodiment of a method of tracking the infusion set 16 through a plurality of infusion processes is shown in Fig. 2. The method of tracking the infusion set 16, in this embodiment of the method, includes operatively coupling the infusion set 16 (which includes the e-tag 18) to the infusion pump 14 (as shown at reference numeral 22). Once the infusion set 16 is coupled to the infusion pump 14, the e-tag 18 is activated. Activation of the e-tag 18 may be accomplished by, for example, 1) electrical connections (if a wired connection exists), 2) a magnetic field, RF, or light transmission (if a wireless connection exists), and/or 3) manual means such as barcode scanning, switches, keypresses/data entry, physical sensing means, and/or the like.

It is to be understood that, prior to coupling the infusion set 16 to the infusion pump 14, the infusion set 16 may be calibrated for use with the infusion pump 14. It is to be understood that rotation of the infusion pump tends to be relatively accurate because the rotation is locked to a crystal time base. The pumping efficacy of the infusion set, however, tends to be more variable. This variance is due, in part, to various factors such as the thickness of the tube wall, the inner diameter of the tube, the durometer of the tube material, the length of the tube, dimensional variance in various components of the infusion set 16, and/or the like. Although infusion pumps currently available tend to assume a value for pumping efficacy based on a measured population of pumps and disposable infusion sets, the addition of an embedded calibration unit in the infusion set 16 would possibly allow a more accurate characterization of the pumping efficacy. For example, such characterization may be done for each individual infusion set, for a group of infusion sets, or for a manufacturing lot of infusion sets. Improvements in medication delivery accuracy in addition to increased design flexibility may be evident in light of using the calibration unit without having to re-program or otherwise adapt the current infusion pump 14 to the infusion set 16 being used.

Data relating to a previous infusion process may be requested by the control element 20 from the e-tag 18 (as shown at reference numeral 24). The data requested includes, e.g., infusion parameters used during the previous infusion process, time elapsed during the previous infusion process, a volume of infusate used during the previous infusion process, and/or combinations thereof. Alternatively or in addition to the data relating to the previous infusion process, the control element 20 may also request the prescribed infusion protocol and parameters. The data and/or the prescribed infusion protocol and parameters are used to perform the instant infusion process (as shown at reference numeral 26).

After the instant infusion process is complete, new data related to the instant infusion process is sent from the control element 20 to the e-tag 18 (as shown at reference numeral 28). The new data includes, e.g., infusion parameters of the instant infusion process, time elapsed during the instant infusion process, a volume of infusate used during the instant infusion process, and/or combinations thereof. In an example, the new data is sent with a request from the control element 20 that the new data be stored in the e-tag 18. The new data is then written to the e-tag 18 (as shown at reference numeral 30). At least the new data may then be used in a subsequent infusion process (as shown at reference numeral 32).

It is to be understood that the terms "connect/connected," "couple/coupled" or the like is broadly defined herein to encompass a variety of divergent connecting or coupling arrangements and assembly techniques. These arrangements and techniques include, but are not limited to (1) the direct connection or coupling between one component and another component with no intervening components therebetween; and (2) the connecting or coupling of one component and another component with one or more components therebetween, provided that the one component being "connected to" or "coupled to" the other component is somehow operatively coupled to the other component (notwithstanding the presence of one or more additional components therebetween).

While several embodiments have been described in detail, it will be apparent to those skilled in the art that the disclosed embodiments may be modified. Therefore, the foregoing description is to be considered exemplary rather than limiting.

## Claims

1. An electronic tag system, comprising:
a medical device having a base portion and a removable portion;
an electronic tag operatively connected to the removable portion, the electronic tag including data related at least to a medicinal infusion process; and
a control element operatively connected to the base portion, the control element including:
a reading capability for reading the data from the electronic tag; and
a writing capability for writing new data to the electronic tag.

2. The electronic tag system as defined in claim 1 wherein the control element further includes a communication means, and wherein the communication means is configured to allow the base portion to communicate with the electronic tag.

3. The electronic tag system as defined in claim 2 wherein the control element, via the communication means, is configured to make a request for at least the data from the electronic tag, to send at least the new data to the electronic tag, or combinations thereof.

4. The electronic tag system as defined in claim 3 wherein the request is at least one of: to perform an encryption of the data; to perform a decryption of the data; to store the new data; to store a program; to send the data; to send a signal representing the data; or combinations thereof.

5. The electronic tag system as defined in claim 3 wherein the request is to send limited access data.

6. The electronic tag system as defined in claim 5 wherein the limited access data is selected from part numbers, serial numbers, lot codes, date codes, manufacturing information, encryption keys, and combinations thereof.

7. The electronic tag system as defined in claim 1 wherein the data includes infusion parameters for performing an instant infusion process, infusion results from a previous infusion process, or combinations thereof.

8. The electronic tag system as defined in claim 1 wherein the new data includes infusion results from an instant infusion process, infusion parameters for a subsequent infusion process, or combinations thereof.

9. A communication method between a pump station and a removable infusion set, the method comprising:
operatively coupling the removable infusion set to the pump station, the removable infusion set including an electronic tag operatively connected thereto;
communicating with the electronic tag via a communication means established with a control element operatively connected to the pump station, the control element including:
a reading capability for reading data from the electronic tag; and
a writing capability for writing at least new data to the electronic tag; and
responding to the communication.

10. The method as defined in claim 9 wherein the communicating with the electronic tag includes making a request from the electronic tag.

11. The method as defined in claim 10 wherein the request is at least one of: to perform an encryption of data stored in the electronic tag; to perform a decryption of the data stored in the electronic tag; to store new data; to store a program; to send stored data; to send a signal representing the stored data; or combinations thereof.

12. The method as defined in claim 11 wherein the request is to send limited access data, and wherein the limited access data is selected from part numbers, serial numbers, lot codes, date codes, manufacturing information, encryption keys, and combinations thereof.

13. A method of tracking a removable infusion set through a plurality of infusion processes, the method comprising:
operatively coupling the removable infusion set to an infusion pump, the removable infusion set including an electronic tag operatively connected thereto;
requesting, from the electronic tag, data related to a previous infusion process, a prescribed infusion process, or a combination thereof, the requesting being accomplished by a control element operatively connected to the infusion pump;
using at least some of the data to perform an instant infusion process;
sending, from the control element to the electronic tag, new data related to the instant infusion process;
writing the new data to the electronic tag; and
using at least the new data in a subsequent infusion process.

14. The method as defined in claim 13 wherein the data related to the previous infusion process includes infusion parameters used during the previous infusion process, time elapsed during the previous infusion process, a volume of infusate used during the previous infusion process, or combinations thereof.

15. The method as defined in claim 13 wherein the new data related to the instant infusion process includes infusion parameters of the instant infusion process, time elapsed during the instant infusion process, a volume of infusate used during the instant infusion process, or combinations thereof.
